# EUROPÄISCHE PATENTSCHRIFT

(11) **EP 4 463 191 B1**
(45) Veröffentlichungstag und Bekanntmachung des Hinweises auf die Patenterteilung: **01.07.2026**
(21) Anmeldenummer: 22817132.8
(22) Anmeldetag: 08.11.2022
(51) Int. Cl.: A61L 15/58, A61L 24/00

(54) **THERMORESPONSIVE WUNDAUFLAGEN**
THERMORESPONSIVE WOUND DRESSING
PANSEMENT THERMOSENSIBLE

(30) Priorität: 11.01.2022 DE 102022100519
(43) Veröffentlichungstag der Anmeldung: 20.11.2024
(73) Patentinhaber: Baden-Württemberg Stiftung gGmbH, 70174 Stuttgart (DE)
(72) Erfinder: STOLZ, Benjamin, 79108 Freiburg (DE); MÜLHAUPT, Rolf, 79117 Freiburg (DE); STEINBERG, Thorsten, 68169 Mannheim (DE)
(74) Vertreter: Müller-Boré & Partner Patentanwälte PartG mbB
(86) Internationale Anmeldenummer: PCT/EP2022/081113
(87) Internationale Veröffentlichungsnummer: WO 2023/134899

(56) Entgegenhaltungen:
- US-A- 2 984 652
- US-A- 5 156 911
- I. ORIENTI ET AL: "Fatty acid substituted polyvinyl alcohol as a supporting material for microsphere preparation", JOURNAL OF MICROENCAPSULATION., vol. 18, no. 1, 1 January 2001 (2001-01-01), GB, pages 77 - 87, XP093025981, ISSN: 0265-2048, DOI: 10.1080/026520401750038629
- CONGDON THOMAS ET AL: "Thermoresponsive, well-defined, poly(vinyl alcohol) co-polymers", POLYMER CHEMISTRY, vol. 6, no. 26, 1 January 2015 (2015-01-01), Cambridge, pages 4749 - 4757, XP093025443, ISSN: 1759-9954, DOI: 10.1039/C5PY00775E

## Beschreibung

Die vorliegende Erfindung betrifft eine thermoresponsive Wundauflage mit thermischschaltbarer Haftung/Enthaftung und ein Verfahren zur Herstellung dieser thermoresponsiven Wundauflage.

Noch heute basiert der Großteil der verwendeten selbstklebenden Wundauflagen auf dem vor über 100 Jahren entwickelten Hansaplast^{®}- bzw. Leukoplast^{®}-System, im Speziellen auf Basis von Zinkoxid-Kautschukkleber. Um das schmerzhafte Entfernen dieser Wundauflagen zu verbessern und das Zurückbleiben von Kleberückständen auf der Wunde zu minimieren, wird nunmehr seit über 30 Jahren an alternativen Klebematerialien auf Basis des "Bond/Debond-on-Command" Prinzips geforscht (vgl. US 5 156 911 A).

Das Funktionsprinzip dieser Materialien besteht darin, dass diese bei Körpertemperatur zuverlässig haften und durch Kühlen oder Erwärmen kontrolliert enthaften. Dieser temperaturinduzierte Übergang von Haftung zu Enthaftung kann derzeit durch zwei Strategien umgesetzt werden. Zum einen kann die schaltbare Klebrigkeit durch einen Kristallisationsprozess erreicht werden. Zum anderen kann das Klebematerial durch thermoreversibles Schalten zwischen hydrophilem und hydrophobem Charakter das gewünschte Verhalten aufweisen.

Die erste Strategie beruht auf thermoresponsiven Polymeren mit kristallisierfähigen Seitenketten, welche bei Raumtemperatur fest und bei Körpertemperatur geschmolzen vorliegen und nur im geschmolzenen Zustand Haftung aufweisen. Durch Kühlen kristallisieren diese Seitenketten, was zum Verlust der Haftung auf der Haut führt. Die Kristallisation bewirkt dabei eine physikalische Vernetzung des Materials, wodurch die Haftung auf der Haut stark gehemmt wird und gleichzeitig die Kohäsion des Materials zunimmt. Dadurch lässt sich die entsprechende Wundauflage an einem Stück ohne Kleberückstände und ohne Schmerzen zu verursachen entfernen. Gleichzeitig kommt es zu einer Volumenverkleinerung, wodurch die Kontaktfläche zwischen Wundauflage und Haut verringert und ein Ablösen der entsprechenden Wundauflage unterstützt wird. Thermoresponsive Polymere, die selbst nicht als Haftkleber für Hautanwendungen geeignet waren, wurden anderen Haftklebern bei Hautanwendungen als Additive zugesetzt (vgl. US 5 156 911 A und R. A. Chivers, International Journal of Adhesion and Adhesives 2001, 21, 381-388). Die Kristallisationstemperatur und die Kristallinität von Vinylesterpolymeren können über die Seitenkettenlänge eingestellt werden (vgl. D. Heinze et al., Thermochimica Acta 2016, 637, 143-153)

US 5 156 911 A beschreibt in diesem Zusammenhang die freie radikalische Copolymerisation von Hexadecylacrylat mit Ethylacrylat im Massenverhältnis von 5:1, was statistische Vinylestercopolymere mit einer Schmelztemperatur von 34 °C liefert, die bei menschlicher Körpertemperatur haften und sich durch Kühlung leicht von der Haut entfernen lassen. Die Ethyl-Ketten des Ethylacrylats sind dabei zu kurz, um kristallisieren zu können, wohingegen die Hexadecyl-Ketten des Hexadecylacrylats beim Kühlen eine Seitenkettenkristallisation einleiten. Dieses Prinzip lässt sich auf andere Acrylat-basierte Systeme übertragen. So führt die freie radikalische Copolymerisation von Pentadecylacrylat und Acrylsäure im Massenverhältnis von 19:1 oder von Hexadecylacrylat, Isodecylacrylat und Acrylsäure im Massenverhältnis von 16:3:1 jeweils zu statistischen Copolymeren mit ähnlichen Eigenschaften.

Die zweite Strategie zum Erhalt von thermoreversibler Klebrigkeit bei Wundauflagen basiert auf einem Wechsel zwischen Hydrophilie und Hydrophobie aufgrund einer unteren kritischen Lösungstemperatur (LCST) oder einer oberen kritischen Lösungstemperatur (UCST). Bei einem LCST-induzierten Wechsel sind unterhalb der LCST intermolekulare Wasserstoffbrückenbindungen zwischen Polymerketten und Wassermolekülen thermodynamisch begünstigt und es dominiert hydrophiles Verhalten. Durch die Temperaturerhöhung über die LCST werden intramolekulare Wechselwirkungen bevorzugt, wodurch das Polymer hydrophob wird. Bei einem UCST-basierten Wechsel zwischen Hydrophilie und Hydrophobie ist das Verhalten umgekehrt, wodurch es zu einer Hydrophobisierung durch Abkühlung unterhalb der UCST kommt (vgl. H. Yamauchi et al., The Journal of Physical Chemistry. B 2007, 111, 12964-12968 und A. Gandhi et al., Asian Journal of Pharmaceutical Sciences 2015, 10, 99-107). Literaturbekannte Beispiele für Materialien mit LCST sind z.B. Hydrogele aus vernetztem Polyvinylalkohol (PVA) als Trägermaterial mit vernetztem Poly-*N*-isopropylacrylamid (PNIPAm). Dabei besitzt PNIPAm eine LCST, welche je nach Verhältnis von PNIPAm zu PVA bei 30 bis 37°C liegt. Für eine Anwendung als Haftmittel einer Wundauflage ist dieses Hydrogel jedoch nicht geeignet, da die LCST oberhalb der menschlichen Körpertemperatur liegen muss (vgl. J.-T. Zhang, et al., Colloid and Polymer Science 2003, 281, 580-583 und A. C. Wenceslau et al., Materials Science and Engineering: C 2012, 32, 1259-1265). Ein LCST basiertes System, was hingegen bereits in Wundauflagen verwendet wird, besteht aus Polypropylen (PP), Chitosan und PNIPAm. Dabei wird zunächst Acrylsäure auf das entsprechende PP gepfropft und anschließend werden, mithilfe von einem Carbodiimid als Kupplungsreagenz, Chitosan und PNIPAm angebunden (vgl. J.-P. Chen et al., Applied Surface Science 2012, 262, 95-101).

Q. Wang et al. zeigten als Beispiel für anwendungstaugliche UCST-basierte Hydrogele bereits eine Kombination aus vernetzter Polyacrylsäure (PAAc) mit vernetztem Polyacrylamid (PAAm). Die UCST des Hydrogels liegt bereits bei 35°C, also an der oberen Grenze des erforderlichen Bereichs. Durch Kühlung kommt es hierbei zur Phasenseparation und das Hydrogel löst sich vom wässrigen Wundbett ab. Zusätzlich konnte gezeigt werden, dass β-Cyclodextrin auf das PAAc-Netzwerk aufgepfropft werden kann, welches der zusätzlichen Arzneimittelfreisetzung dienen kann (Q. Wang et al., Journal of Applied Polymer Science 2009, 111, 1417-1425).

Neben dem temperaturinduzierten "Bond/Debond-on-Command" gibt es die Möglichkeit, durch UV-Einwirkung die Haftung und Enthaftung von Klebematerialien zu steuern. Hierbei bildet sich bei der Bestrahlung durch photochemische Reaktion ein kovalentes Netzwerk mit deutlich reduzierter Haftung aus. Als Klebematerial für Wundauflagen sind diese Systeme jedoch nicht geeignet, da der Einsatz photosensitiver Bestandteile wie Epoxyacrylate, Photoinitiatioren oder Übergangsmetallkomplexe bei Wundkontakt als kritisch zu bewerten sind (W. Zhang et al., Journal of Applied Polymer Science 2018, 135, 46435).

Es lässt sich damit festhalten, dass diverse Klebematerialien auf Basis des "Bond/Debond-on-Command" Prinzips bereits zum Stand der Technik gehören. Jedoch ist ebenfalls ersichtlich, dass weiterhin Verbesserungsbedarf für solche Klebematerialien hinsichtlich (i) des Zurückbleibens von Kleberückständen am Patienten beim schmerzfreien Entfernen dieser und (ii) Biokompatibilität besteht. Insbesondere bei der stationären Wundbehandlung von Patienten können Haftmittelrückstande zu Komplikationen mit der entsprechenden Hautverletzung führen und müssen vom Pflegepersonal aufwendig entfernt werden.

Demgemäß ist eine Aufgabe der vorliegenden Erfindung, thermoresponsive Wundauflage, umfassend entsprechende Haftmittel-Zusammensetzungen, bereitzustellen, welche ein schmerzfreies Entfernen für den Patienten gewährleisten, wobei das Zurückbleiben von Rückständen der Haftmittel-Zusammensetzung am Patienten minimiert ist und die zudem auch eine exzellente Biokompatibilität aufweisen.

Die vorstehend beschriebene Aufgabe wird durch die in den Ansprüchen gekennzeichneten Ausführungsformen der vorliegenden Erfindung gelöst.

Insbesondere werden erfindungsgemäß eine thermoresponsive Wundauflage mit thermisch schaltbarer Haftung/Enthaftung bereitgestellt, umfassend:
ein Trägermaterial, und
eine Haftmittel-Zusammensetzung, aufgebracht auf einer Oberfläche des Trägermaterials, wobei die Haftmittel-Zusammensetzung umfasst:
   mindestens ein Vinylalkohol/Copolymer mit der nachstehenden Formel (I): 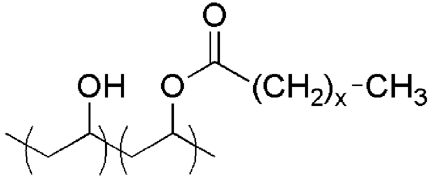
   wobei x bezüglich jeder Fettsäureeinheit unabhängig voneinander eine ganze Zahl von 10 bis 18 beträgt,
   wobei das Copolymer gemäß Formel (I) bei Körpertemperatur als Schmelze vorliegt, die auf Wunden und Haut haftet, und beim Abkühlen auf eine Temperatur oberhalb des Gefrierpunkts von Wasser die Kristallisation des Copolymers gemäß Formel (I) die Enthaftung der Wundauflage bewirkt.

Der Einsatz der vorstehend definierten Haftmittel-Zusammensetzung führt vorteilhafterweise dazu, dass die erfindungsgemäßen thermoresponsiven Wundauflagen nach Gebrauch schmerzfrei und mit minimierten Rückständen vom Patienten entfernt werden können und die erfindungsgemäßen thermoresponsiven Wundauflagen zudem eine exzellente Biokompatibilität aufweisen. In diesem Zusammenhang wurde überraschend entdeckt, dass sich Fettsäureester von Polyvinylalkohol durch thermisch schaltbare Haftung/Enthaftung und rückstandsfreie Enthaftung auszeichnen.

Dabei bedeutet der Begriff "thermoresponsiv" gemäß der vorliegenden Erfindung, dass sich die physikalischen Eigenschaften des mindestens einen Copolymers, dargestellt durch die Formel (I), drastisch und diskontinuierlich mit der Temperatur ändern, insbesondere einen thermisch schaltbaren Phasenübergang zwischen kristallin und amorph unterhalb von Körpertemperatur und oberhalb des Gefrierpunktes von Wasser aufweisen.

Insbesondere wenn das mindestens eine Copolymer mit der Formel (I) eine Schmelztemperatur (Tₘ) von 20 bis 35°C und eine Schmelzenthalpie (ΔHₘ) von 20 J/g oder mehr aufweist, liegt das Copolymer gemäß Formel (I) bei Körpertemperatur als auf Wunden und Haut gut haftende Schmelze vor. Beim Vorliegen eines Copolymers gemäß Formel (I) mit einer solchen Schmelztemperatur und einer solchen Schmelzenthalpie bewirkt dann beim Abkühlen auf eine Temperatur oberhalb des Gefrierpunkts von Wasser die Kristallisation des Copolymers gemäß Formel (I) die Enthaftung der Wundauflage.

Das vorstehend definierte mindestens eine Copolymer mit der Formel (I) weist bevorzugt eine Schmelztemperatur (Tₘ) von 22 bis 33°C, mehr bevorzugt von 24 bis 31°C, besonders bevorzugt von 26 bis 29°C auf. Beispielsweise kann das mindestens eine Copolymer mit der Formel (I) eine Schmelztemperatur (Tₘ) von 20 bis 33°C, 20 bis 31°C, 20 bis 29°C, 22 bis 35°C, 22 bis 31°C, 22 bis 29°C, 24 bis 35°C, 24 bis 33°C, 24 bis 29°C, 26 bis 35°C, 26 bis 33°C oder 26 bis 31°C aufweisen.

Weiterhin weist das vorstehend definierte, mindestens eine Copolymer mit der Formel (I) bevorzugt eine Schmelzenthalpie (ΔHₘ) von 25 J/g oder mehr, mehr bevorzugt von 30 J/g oder mehr, besonders bevorzugt von 35 J/g oder mehr, auf.

Die Schmelztemperatur (Tₘ) und die Schmelzenthalpie (ΔHₘ) des vorstehend definierten mindestens einen Copolymers mit der Formel (I) können beispielsweise mittels DSC (differential scanning calorimetry) bei einer Heizrate von 10 K/min bestimmt werden.

Es ist festzuhalten, dass die erfindungsgemäß eingesetzten PVA/Fettsäurester-Copolymere mit dem in US 5 156 911 A beschriebenen Verfahren nicht zugänglich sind. So kann beispielsweise durch das Verestern von Polyvinylakohol mit Fettsäuren über die Seitenkettenkristallisation die Schmelz- und Kristallisationstemperatur unterhalb von Körpertemperatur gezielt eingestellt werden. Polyvinylalkohol ist biokompatibel und gut hautverträglich, weist aber nur eine Hauptkettenkristallisation bei Temperaturen weit oberhalb der Körpertemperatur auf. Während bei der Hydrolyse der erfindungsgemäß eingesetzten PVA/Fettsäurester-Copolymere in vorteilhafter Weise hautverträglicher Polyvinylakohol gebildet wird, entsteht durch die Hydrolyse bei den in US 5 156 911 A beschriebenen Vinylestercopolymeren bzw. (Meth)Acrylatcopolymeren hautreizende Poly(meth)acrylsäure. Zudem kann über die molekularen Architekturen der erfindungsgemäß eingesetzten PVA/Fettsäurester-Copolymere das rückstandslose Ablösen erzielt werden.

Erfindungsgemäß ist die vorstehend genannte Haftmittel-Zusammensetzung für die erfindungsgemäßen thermoresponsiven Wundauflagen nicht weiter beschränkt, sofern diese mindestens ein Copolymer mit der vorstehend dargestellten Formel (I) umfasst. Das Copolymer kann ein statistisches Copolymer, ein Gradientenpolymer oder ein Blockpolymer, quasi aufgebaut aus Vinylalkohol-Einheiten und Vinylfettsäureester-Einheiten, sein. Vorzugsweise, insbesondere im Lichte der einfacheren Herstellung mittels Veresterung von Polyvinylakohol mit Fettsäuren, ist das Copolymer gemäß Formel (I) ein statistisches Copolymer. Die Haftmittel-Zusammensetzung kann dabei ein statistisches Copolymer oder zwei oder mehr statistische Copolymere mit der vorstehend genannten Formel (I) umfassen, wobei die zwei oder mehr statistischen Copolymere verschieden voneinander sind.

Vorzugsweise ist das molare Verhältnis von Vinylalkohol-Einheiten zu Vinylfettsäureester-Einheiten 0,02 bis 0,80, bevorzugt 0,03 bis 0,70, mehr bevorzugt 0,04 bis 0,60, besonders bevorzugt 0,05 bis 0,50. Beispielsweise kann das molare Verhältnis von Vinylalkohol-Einheiten zu Vinylfettsäureester-Einheiten 0,02 bis 0,70, 0,02 bis 0,60, 0,02 bis 0,50, 0,03 bis 0,80, 0,03 bis 0,60, 0,03 bis 0,50, 0,04 bis 0,80, 0,04 bis 0,70, 0,04 bis 0,50, 0,05 bis 0,80, 0,05 bis 0,70, oder 0,05 bis 0,60 sein.

In einer besonders bevorzugten Ausführungsform der vorliegenden Erfindung beträgt x gleich 12 und das molare Verhältnis von Vinylalkohol-Einheiten zu Vinylfettsäureester-Einheiten 0,05 bis 0,50.

Das Verhältnis von Vinylalkohol-Einheiten zu Vinylfettsäureester-Einheiten des mindestens einen, vorzugsweise statistischen, Copolymers mit der vorstehend dargestellten Formel (I) kann mittels ¹H-NMR-Spektroskopie in beispielsweise CDCl₃ als Lösungsmittel ermittelt werden. Dass es sich vorzugsweise um ein statistisches Copolymer der vorstehend dargestellten Formel (I) handelt, anstatt beispielsweise um zwei Homopolymere der jeweilig verwendeten Monomere, kann mittels 2D-DOSY-NMR-Spektroskopie in beispielsweise CDCl₃ bestimmt werden. Bei erfolgreicher statistischer Copolymerisation der jeweiligen Monomere weisen alle Signale denselben Diffusionskoeffizienten in dem jeweils verwendeten deuterierten Lösungsmittel, beispielsweise CDCl₃, auf.

Das mindestens eine Copolymer mit der vorstehend dargestellten Formel (I) kann gegebenenfalls auch ein oder mehrere, weitere Einheiten, abgeleitet von Monomeren, ausgewählt aus Ethylen und Vinylacetat, umfassen, und zwar in Anteilen, so lange nicht die thermoresponsiven Eigenschaften des Copolymers nachteilig beeinträchtigt werden.

In einer weiteren Ausführungsform weist das mindestens eine Copolymer eine zahlengemittelte Molmasse (Mₙ) von 1000 bis 100000 g/mol, bevorzugt 3000 bis 90000 g/mol, mehr bevorzugt 5000 bis 80000 g/mol, besonders bevorzugt 10000 bis 70000 g/mol auf. Beispielsweise kann das mindestens eine Copolymer gemäß Formel (I) ein Mₙ von 1000 bis 90000 g/mol, 1000 bis 80000 g/mol, 1000 bis 70000 g/mol, 3000 bis 100000 g/mol, 3000 bis 80000 g/mol, 3000 bis 70000 g/mol, 5000 bis 100000 g/mol, 5000 bis 90000 g/mol, 5000 bis 70000 g/mol, 10000 bis 100000 g/mol, 10000 bis 90000 g/mol oder 10000 bis 80000 g/mol aufweisen.

Der Polydispersitätsindex (PDI) des mindestens einen Copolymers ist nicht weiter beschränkt und kann beispielsweise 1,00 bis 3,00, 1,00 bis 2,50, 1,00 bis 2,00 oder 1,00 bis 1,50 betragen. Der PDI entspricht hierbei der Breite der Häufigkeitsverteilung einzelner Molmassen.

Diverse Methodiken zur Bestimmung des Mₙ und PDI eines Copolymers sind dem Fachmann bekannt, wie beispielsweise MALDI-ToF-Massenspektrometrie, ESI-ToF-Massenspektrometrie, Gel-Permeations-Chromatographie (GPC, SEC), Assymmetrische-Fluss-Feldflussfraktionierung (AF4), Dampfdruckosmometrie und ¹H-NMR-Spektroskopie. Das Mₙ und der PDI des mindestens einen statistischen Copolymers können beispielsweise durch Gel-Permeations-Chromatographie (GPC) bestimmt werden.

In einer weiteren Ausführungsform umfasst die Haftmittel-Zusammensetzung das mindestens eine Copolymer in einer Menge von 40 bis 100 Gewichts-%, bevorzugt 50 bis 99 Gewichts-%, mehr bevorzugt 60 bis 95 Gewichts-%, besonders bevorzugt 70 bis 90 Gewichts-%. Beispielsweise kann die Haftmittel-Zusammensetzung das mindestens eine Copolymer in einer Menge von 40 bis 99 Gewichts-%, 40 bis 95 Gewichts-%, 40 bis 90 Gewichts-%, 50 bis 100 Gewichts-%, 50 bis 95 Gewichts-%, 50 bis 90 Gewichts-%, 60 bis 100 Gewichts-%, 60 bis 99 Gewichts-%, 60 bis 90 Gewichts-%, 70 bis 100 Gewichts-%, 70 bis 99 Gewichts-% oder 70 bis 95 Gewichts-% umfassen.

In einer bevorzugten Ausführungsform der vorstehend definierten Haftmittel-Zusammensetzung liegt, unterhalb der Schmelztemperatur (Tₘ) des mindestens einen Copolymers, die Seitenketten-Kristallinität des mindestens einen Copolymers in Form von einer "end-on"-Kristallstruktur, besonders bevorzugt ausschließlich in Form von einer "end-on"-Kristallstruktur, vor.

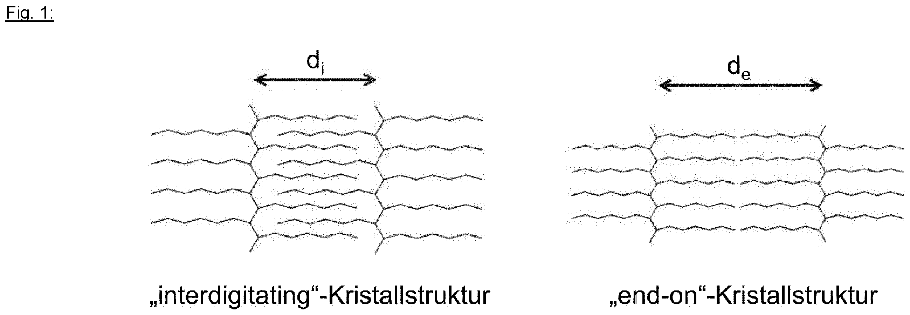

Im Falle einer Seitenkettenkristallisation eines Polymers existieren zwei verschiedene Kristallisationsmechanismen, nach welchen sich die Seitenketten zu Kristalliten anlagern können. Je nach Packungsart wird die zugrunde liegende Kristallstruktur als "end-on"- oder "interdigitating"-Kristallstruktur bezeichnet, wie in Figur 1 schematisch dargestellt. Im Falle der dichter gepackten "interdigitating"-Struktur ragen die Seitenketten in die Zwischenräume der Seitenketten der Nachbarmoleküle und liegen somit parallel zu den benachbarten Seitenketten vor. Im Gegensatz dazu sind die Seitenketten in der "end-on"-Kristallstruktur frontal zu den Seitenketten der Nachbarmoleküle orientiert.

Die Kristallitdicke (d) ist im Falle der "interdigitating"-Kristallstruktur somit wesentlich kleiner als im Falle der "end-on"-Kristallstruktur. Im ideal gepackten Fall entspricht die Kristallitdicke für die "interdigitating"-Kristallstruktur (dᵢ) der Länge der Seitenkette. Die Kristallitdicke in der ideal gepackten "end-on"-Kristallstruktur (dₑ) entspricht der doppelten Länge der Seitenkette zuzüglich eines C-C Abstands zwischen den beiden Enden der frontal gepackten Seitenketten.

Wenn das vorstehend definierte mindestens eine Copolymer mit der Formel (I) eine Schmelztemperatur (Tₘ) von 20 bis 35°C und eine Schmelzenthalpie (ΔHₘ) von 20 J/g oder mehr aufweist, weist die erfindungsgemäße thermoresponsive Wundauflage eine zuverlässige Schälfestigkeit bei 0 bis 37°C an der Haut des entsprechenden Patienten auf.

Die Schälfestigkeit der erfindungsgemäßen thermoresponsiven Wundauflage, umfassend solche Haftmittel-Zusammensetzungen wie vorstehend beschrieben, beträgt bei 0 bis 37°C 0,30 bis 8,00 N/10 mm, bevorzugt 0,40 bis 7,50 N/10 mm, mehr bevorzugt 0,60 bis 6,00 N/10 mm, besonders bevorzugt 0,80 bis 5,50 N/10 mm. Beispielsweise kann die Schälfestigkeit bei 0 bis 37°C 0,30 bis 7,50 N/10 mm, 0,30 bis 6,00 N/10 mm, 0,30 bis 5,50 N/10 mm, 0,40 bis 8,00 N/10 mm, 0,40 bis 6,00 N/10 mm, 0,40 bis 5,50 N/10 mm, 0,60 bis 8,00 N/10 mm, 0,60 bis 7,50 N/10 mm, 0,60 bis 5,50 N/10 mm, 0,80 bis 8,00 N/10 mm, 0,80 bis 7,50 N/10 mm oder 0,80 bis 6,00 N/10 mm betragen.

Die Schälfestigkeit der erfindungsgemäßen thermoresponsiven Wundauflage kann bei 0 bis 37°C mittels eines 180° Abzugstests nach "ASTM D3330 Test Method A 180 Degree Peel Test" bestimmt werden.

Wenn das vorstehend definierte, mindestens eine Copolymer mit der Formel (I) eine Schmelztemperatur (Tₘ) von 20 bis 35°C und eine Schmelzenthalpie (ΔHₘ) von 20 J/g oder mehr aufweist, ermöglicht die erfindungsgemäße thermoresponsive Wundauflage nach Kühlung ein für den Patienten schmerzfreies und rückstandsloses Entfernen der Wundauflage von der Haut des Patienten.

Zudem weist das vorstehend definierte Copolymer mit der Formel (I) eine exzellente Biokompatibilität auf. Im Speziellen entstehen durch etwaige Hydrolyse des vorstehend dargestellten Copolymers mit der Formel (I) lediglich Polyvinylalkohol (PVA) und Fettsäuren, welche medizinisch als unbedenklich gelten.

Die Biokompatibilität des erfindungsgemäß eingesetzten Copolymers gemäß Formel (I) kann nach ISO 10993-05 evaluiert werden.

Bevorzugt kann die vorstehend definierte Haftmittel-Zusammensetzung weiter mindestens eine antimikrobielle Substanz umfassen. Folglich kann die vorstehend definierte Haftmittel-Zusammensetzung eine antimikrobielle Substanz oder zwei oder mehr antimikrobielle Substanzen umfassen.

Antimikrobielle Substanzen sind hierbei als chemische Stoffe zu verstehen, welche die Vermehrungsfähigkeit oder Infektiosität von Mikroorganismen, z.B. Bakterien und Viren, reduzieren oder sie unschädlich machen bzw. inaktivieren. Erfindungsgemäß sind die antimikrobiellen Substanzen nicht weiter beschränkt, sofern diese antimikrobielle Wirkung entfalten. Antimikrobielle Substanzen für Wundauflagen sind bereits bekannt und können zum Beispiel Chlorhexidin, Thymol, Eugenol, Chlorphenole, Silberionenhaltige Phosphorsäuresalze, Alginate, Chlordiphenylether oder natürliche oder synthetische Zeolithe, die Silber, Kupfer oder Zink enthalten, sein.

Bevorzugt umfasst die vorstehend definierte Haftmittel-Zusammensetzung weiter mindestens eine antimikrobielle Substanz in einer Menge von 1 bis 30 Gewichts-%, mehr bevorzugt 3 bis 25 Gewichts-%, besonders bevorzugt 5 bis 20 Gewichts-%. Beispielsweise kann die vorstehend definierte Haftmittel-Zusammensetzung mindestens eine mikrobielle Substanz in einer Menge von 1 bis 25 Gewichts-%, 1 bis 20 Gewichts-%, 3 bis 30 Gewichts-%, 3 bis 20 Gewichts-%, 5 bis 30 Gewichts-% oder 5 bis 25 Gewichts-% umfassen.

Die vorstehend definierte Haftmittel-Zusammensetzung kann weiter Klebrigmacher, wie beispielsweise Kolophonium oder Polyester, Antioxidantien, wie beispielsweise Vitamin E, faserige oder nichtfaserige Füllstoffe, Farbstoffe oder Kombinationen davon enthalten.

Das vorstehend mindestens eine Copolymer mit der Formel (I) kann beispielsweise durch nachstehende Eliminierungs-Reaktion hergestellt werden: 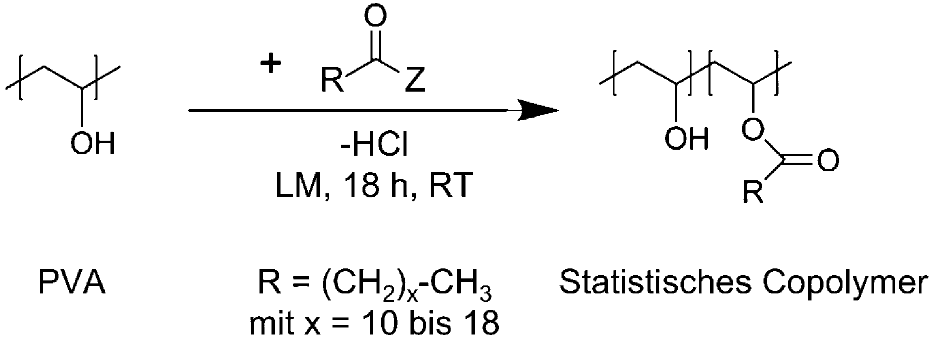 wobei LM für Lösungsmittel und RT für Raumtemperatur steht. Als Lösungsmittel können beispielsweise N-Methyl-2-pyrrolidon (NMP), N-Methylcaprolactam, N-Vinyl-2-pyrrolidon, N-Methylimidazol, N-Methylimidazoliumchlorid, Kresol, Eugenol, ionische Flüssigkeiten oder Gemische davon verwendet werden. PVA kann hierbei beispielsweise Polyvinylalkohol, ein vollständig oder partiell hydrolysiertes Polyvinylacetat oder ein vollständig oder partiell hydrolysiertes Ethylen/Vinylacetat-Copolymer sein. Die vorstehend genannte Eliminierungsreaktion kann jedoch auch in einer Schmelze ohne Lösungsmittel LM durchgeführt werden. Z kann beispielsweise Fluor, Chlor, Brom, Iod, Phenolat, Chlorphenolat, Nitrophenolat, Eugenolat und/oder Glycerin sein.

Das Verhältnis von Vinylalkohol-Einheiten zu Vinylfettsäureester-Einheiten, des vorstehend mindestens einen Copolymers mit der Formel (I) kann hierbei durch Variation des Stoffmengenverhältnisses [PVA]/[Alkylsäure-R'] entsprechend eingestellt werden. Nach Beendigung der vorstehend dargestellten Eliminierungs-Reaktion kann das resultierende Copolymer als Rohprodukt durch einmaliges oder mehrmaliges Lösen und Ausfällen in entsprechend geeigneten Lösungs- und Fällungsmitteln sowie durch Extraktion und Dialyse weiter aufgereinigt werden.

Alternativ kann auch das entsprechende Vinylfettsäurecarboxylat, beispielsweise Vinylmyristinsäurecarboxylat, homopolymerisiert oder mit Vinylacetat copolymerisiert werden, woraus dann vollständig veresterte Polyvinylalkohole resultieren. Durch Umesterung mit mono- oder polyfunktionellen Alkoholen können dann Vinylalkoholeinheiten gebildet werden.

Werden Polyvinylalkohol oder partiell hydrolysierte Polyvinylacetate bzw. entsprechende Ethylen/Vinylacetat (EVA) Copolymere, die entweder vollständig oder partiell hydrolysiert sind, als Ausgangsmaterial mit entsprechendem Vinylfettsäurecarboxylat, beispielsweise Myristinsäuremethylester, verwendet, kann das tiefer siedende Nebenprodukt Methylacetat in vorteilhafter Weise leichter abgetrennt werden, um somit die Handhabung von Säurechloriden als Reaktionspartner zu vermeiden.

Das Trägermaterial der erfindungsgemäßen thermoresponsiven Wundauflage ist nicht weiter beschränkt, sofern die vorstehend definierte Haftmittel-Zusammensetzung darauf aufgebracht werden kann.

Bevorzugt besteht das Trägermaterial der erfindungsgemäßen thermoresponsiven Wundauflagen aus mindestens einem Material, ausgewählt aus der Gruppe, bestehend aus Cellulose, Polyvinylchlorid, Polyethylen, Polyethylenterephthalat, Polyurethan und Polyetherester.

Die erfindungsgemäße thermoresponsive Wundauflage kann durch Aufbringen einer vorstehend definierten Haftmittel-Zusammensetzung auf eine der Oberflächen des Trägerelements hergestellt werden. Das Aufbringen ist dabei nicht weiter beschränkt, sofern die vorstehende Haftmittel-Zusammensetzung auf eine der Oberflächen des Trägerelements aufgebracht wird. Das Aufbringen der vorstehenden Haftmittel-Zusammensetzung auf eine der Oberflächen des Trägerelements kann beispielsweise durch Beschichten, Sprayen, 3D-Drucken, Aufrollen oder Eintauchen realisiert werden. Das Beschichten kann mit Schmelzen, Lösungen oder Dispersionen von einem oder mehreren der vorstehenden Copolymere gemäß Formel (I) erfolgen.

Die Figuren zeigen:
Fig. 1 zeigt mögliche Kristallstrukturen von Polymeren durch Seitenkettenkristallisation: "interdigitating"-Kristallstruktur (links); "end-on"-Kristallstruktur (rechts).
Fig. 2 zeigt den Versuchsaufbau bei 180° Abzugstests nach "ASTM D3330 Test Method A 180 Degree Peel Test": Wundauflage gemäß der vorliegenden Erfindung (links); Leukoplast^{®}-Referenz (rechts).
Fig. 3 zeigt links: SAXS-Diffraktogramme der Beispiele 1 und 2; rechts: Abstand zweier Kohlenstoffatome entlang einer Alkylseitenkette unter Annahme von 0,153 nm Bindungslänge und Tetraederwinkel.
Fig. 4 zeigt die Schälfestigkeit in N/10 mm der Beispiele 1 und 2 bei 37°C und 0°C im Vergleich zu einem Leukoplast^{®}-Tape.
Fig. 5 zeigt die Auftragung des Cell Index gegen die Zeit als Maß für das Zellwachstum im Eluattest des Beispiels 1 im Vergleich zu einer Referenzprobe.

### Beispiele

Die folgenden Beispiele dienen der Veranschaulichung der vorliegenden Erfindung, ohne jedoch hierauf beschränkt zu sein.

### a) Verwendete Materialien und Chemikalien:

Azo-*bis*-(isobutyronitril) (AIBN, ≥ 98%), Isopropanol (≥ 99.9%), Vinylstearat (≥ 99.9%, stabilisiert mit Mequinol), Polyvinylalkohol (PVA, Mowiol 6-98, 98% Hydrolysegrad, Mₙ = 47000 g/mol), Palmitoylchlorid (98%), Myristoylchlorid (97%) und Lauroylchlorid (98%) stammen von Sigma-Aldrich Chemie GmbH. Vinylpalmitat (96%, stabilisiert mit Mequinol) wurde von abcr GmbH bezogen. Toluol (≥ 99.9%) und *N*-Methyl-2-pyrollidon (NMP, ≥ 99.8%) stammten von Carl Roth GmbH & Co. KG. Ethanol (EtOH, ≥ 99.9%), Chloroform (≥ 99.98%) und Dimethylsulfoxid (DMSO, ≥ 99,9%) wurden von Fisher Chemical erworben. Diethylether (≥ 99,9%) stammt von VWR. Deuteriertes Chloroform (CDCl3, ≥ 99,8%) wurde von Eurisotop GmbH bezogen. Aceton (techn.) und Methanol (MeOH, techn.) stammen von Oelfabrik Schmidt GmbH. Die Wundauflagen, welche zur Probenpräparation verwendet wurden, waren Zemuko^{®} Vliesstoff-Kompressen (PZN: 01226396).

### b) Charakterisierungsmethoden:

### DSC (differential scanning calorimetry)

Die Bestimmung der Schmelztemperatur (Tₘ) und der Schmelzenthalpie (ΔHₘ) der statistischen Copolymere erfolgte mittels DSC mit Hilfe eines "DSC 204 F1 Phoenix" der Firma Netzsch Gerätebau GmbH. Die Proben wurden von Raumtemperatur auf -50°C gekühlt, danach auf +100°C erhitzt, erneut auf -50°C gekühlt und ein weiteres Mal auf +100°C erhitzt. Die Heizrate betrug in allen Fällen 10 K/min. Vor Beginn jeder Aufheiz- oder Abkühlphase wurde die Probe 5 min auf der jeweiligen Starttemperatur gehalten. Zur finalen Auswertung wurde die Software "Netzsch Proteus 6.1" verwendet.

### Kleinwinkel-Röntgenstreuung (SAXS)

Die SAXS-Experimente erfolgten bei Raumtemperatur mit einer evakuierten Kratky Kompakt Kamera mit Strichfokus (Anton Paar, Graz, Österreich) und ortsempfindlichem Detektor (Hecus-mBraun, Graz, Österreich). Als Röntgenquelle diente eine Röntgenröhre mit Kupferanode, Wellenlänge λ = 0.1542 nm, s = (2/λ) sinθ. Die Lorentz-korrigierten Streukurven wurden final nach Abzug des Untergrunds grafisch aufgetragen.

### Schälfestigkeit

Zur Untersuchung der Schälfestigkeit der thermoresponsiven Wundauflagen wurden 180° Abzugstests nach der Normvorschrift "ASTM D3330 Test Method A 180 Degree Peel Test" durchgeführt. Die Messung erfolgte mittels einer Zwick-Prüfmaschine des Typs Z-005. Der Abstand der beiden Spannköpfe betrug 100 mm und die Standardkraft wurde mit einer 100 N-Messdose aufgenommen. Die Messgeschwindigkeit betrug 5 mm/s. Zur finalen Datenauswertung wurde die "Zwick Test Xpert Software Version 11.0" verwendet.

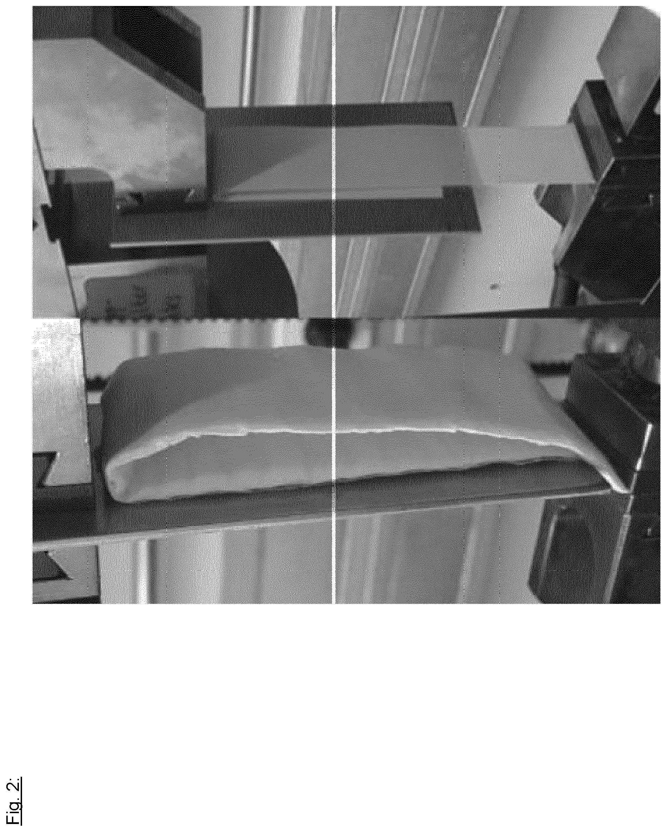

Für Abzugsversuche nach der "ASTM D3330 Test Method A 180 Degree Peel Test" Norm wurden Probenkörper (Maße: 200 x 30 mm) aus einer Zemuko^{®}-Wundauflage zugeschnitten und mit einem Spatel auf einer definierten Klebefläche (Maße: 80 x 15 mm) von mit je 600 mg aufgeschmolzener Haftmittel-Zusammensetzung gleichmäßig bestrichen. Die präparierten Wundauflagen wurden auf einer Edelstahlplatte (Maße: 125 x 50 x 2 mm) unter Beschwerung mit einer Messingplatte (m = 2800 g) auf einem beheizbaren Aluminiumblock (Maße: 15 x 15 x 3 cm) für 5 min bei 37°C getempert. Für die Versuche bei 0°C wurden die Probenkörper anschließend für 120 s mit Eis gekühlt. Für die Versuche bei 37°C wurden die Proben direkt nach dem Tempern vermessen. Um die Reproduktion der 180° Zugversuche zu erleichtern, zeigt Figur 2 den Versuchsaufbau anhand einer präparierten Wundauflage und anhand der Leukoplast^{®}-Referenz. Im oberen Spannkopf der Zug/Dehnungs-Maschine ist die Metallplatte, auf der die Wundauflage aufgeklebt wurde, eingespannt. Im unteren Spannkopf ist das Endstück der Wundkompresse eingespannt, welche oberhalb der Klebefläche um 180° umgeschlagen wurde.

### Rückstand der Haftmittel-Zusammensetzung nach Entfernen der Wundauflagen

Für die Abzugstests wurden Probenkörper (Maße: 60 x 30 mm) aus einer Zemuko^{®} Wundauflage geschnitten und mit einem Spatel mit 300 mg aufgeschmolzener Haftmittel-Zusammensetzung gleichmäßig auf einer definierten Fläche (Maße: 40 x 15 mm) bestrichen. Die präparierten Wundauflagen wurden auf einem beheizbaren Aluminiumblock (Maße: 15 x 15 x 3 cm) unter Belastung mit einer Messingplatte (m = 2800 g) für 5 min bei 37°C getempert. Anschließend wurde der Aluminiumblock für 120 s auf Eis mit 0°C gestellt. Im Anschluss wurden die Wundauflagen mechanisch vom Aluminiumblock nach "ASTM D3330 Test Method A 180 Degree Peel Test" abgezogen und über das Wiegen der Wundauflage vor und nach dem Abzugstest der Kleberückstand ermittelt. Für jede Haftmittel-Zusammensetzung wurden 5 Proben untersucht. Der angegebene Fehler entspricht der Standardabweichung.

### Biokompatibilitätstest

Zur Beurteilung der Biokompatibilität der thermoresponsiven Wundauflagen wurden Eluattests durchgeführt um die Freisetzung zellwachstumshemmender Substanzen auszuschließen. Dazu wurden jeweils 200 mg der zu untersuchenden Proben in einen "E Plate 8 Behälter" gegeben und jeweils 500 µL Zellkulturmedium (HPV 16 E6/E7-immortalisierte Keratinocyte Growth Medium 2 der Firma Promocell) gegeben. Bei den Zellen, die im Medium angesiedelt wurden, handelt es sich um Keratinocyten aus der Mundschleimhaut (HaCaT Zellen der Firma CLS Cell Lines Service). Die Messungen der Impedanz, über welche der Cell Index als Maß für das Zellwachstum berechnet werden konnte, erfolgte anhand des Geräts iCelligence der Firma ACEA Biosciences.

### c) Herstellung der Copolymere:

### Synthese von statistischen Vinylalkohol/Vinylalkylester-Copolymeren

Zur Synthese der statistischen Vinylalkohol/Vinylalkylester-Copolymere wurde unter Stickstoff-Atmosphäre PVA (2,60 g, 59,0 mmol) bei 110°C in NMP (40,0 mL) gelöst. Anschließend wurde bei RT das jeweilige Alkylsäurechlorid zugetropft. Das Reaktionsgemisch wurde für 18 h bei RT gerührt, bevor das Rohprodukt durch Zugabe von entionisiertem Wasser gefällt, abfiltriert und mit Wasser gewaschen wurde. Das Rohprodukt wurde anschließend durch mehrmaliges Lösen und Fällen in geeigneten Lösungs- und Fällungsmitteln aufgereinigt, bis NMP als Lösungsmittel im ¹H-NMR Spektrum (CDCl₃) nicht mehr zu detektieren war. Anschließend wurden die erhaltenen Produkte für 18 h bei 60°C im Vakuumtrockenschrank getrocknet. In Tabelle 1 ist hierzu eine Zusammenfassung der eingesetzten Mengen an Edukten sowie der für die Aufreinigung eingesetzten Lösungs- und Fällungsmittel von zwei beispielhaften Ansätzen aufgelistet.

**Tabelle 1:**

| **Beispiel** | **PVA (mmol)** | **Myristoylchlorid (mmol)** | **Lösungsmittel** | **Fällungsmittel** |
|---|---|---|---|---|
| Beispiel 1 | 59,0 | 44,4 | CHCl₃ | MeOH |
| Beispiel 2 | 59,0 | 59,2 | CHCl₃ | MeOH |

### d) Eigenschaften der Copolymere:

### Schmelztemperatur (Tₘ) und Schmelzenthalpie (ΔHₘ)

Die erhaltenen statistischen Copolymere aus den Beispielen 1 und 2 wurden anschließend hinsichtlich a) des tatsächlichen Veresterungsgrades in Formel (I), b) Schmelztemperatur (Tₘ) und c) Schmelzenthalpie (ΔHₘ) untersucht. Die erhaltenen Ergebnisse sind in Tabelle 2 zusammengefasst.

**Tabelle 2:**

| **Beispiel** | **x (Formel (I))** | **Verhältnis Vinylalkohol-Einheiten/Vinylfettsäureester-Einheiten (Formel (I))** | **Tₘ (°C)** | **ΔHₘ (J/g)** |
|---|---|---|---|---|
| Beispiel 1 | 12 | 0,38 | 26-29 | 35,9 |
| Beispiel 2 | 12 | 0,06 | 22-25 | 40,6 |

In den DSC-Messungen der hergestellten statistischen Vinylalkohol/Vinylalkylester-Copolymere zeigte sich, dass das Schmelzverhalten der Materialien durch die Zusammensetzung gesteuert werden kann. Das Ausgangsmaterial PVA schmilzt bei 217°C. Durch die Veresterung der Alkoholgruppen des PVAs wird die Kristallisation des PVA-Polymerrückgrats vollständig unterbunden und der Einfluss der Seitenkettenlänge und der Seitenkettenkonzentration dominieren das Schmelzverhalten. Es wurde durch die Erfinder festgestellt, dass je höher der Veresterungsgrad und je länger die Seitenkette ist, desto größer folglich auch die Schmelzenthalpie (ΔHₘ) ist. Die vorstehenden Beispiele 1 und 2 erfüllen dabei alle geforderten Bedingungen für die thermischen Eigenschaften eines "bond/debond-on-command"-Materials und somit für die Anwendung als Haftmittel-Zusammensetzungen für thermoresponsive Wundauflagen.

### Seitenketten-Kristallisation

In den Ergebnissen zu den thermischen Eigenschaften der statistischen Copolymere aus den DSC-Messungen zeigte sich ein starker Einfluss auf das Schmelzverhalten durch die Länge und Konzentration der verschiedenen Fettsäureseitenketten. Dies steht im Einklang mit der Annahme, dass das Aufschmelzen und Kristallisieren des Materials durch eine Seitenkettenkristallisation verursacht wird.

Um diese Hypothese weiter zu stützen, wurden Röntgenbeugungsexperimente durchgeführt. Im SAXS (small angle x-ray scattering) Experiment können Überstrukturen von Kristalliten im Material bestimmt werden. Im Falle einer Seitenkettenkristallisation existieren zwei verschiedene Kristallisationsmechanismen, nach welchen sich die Seitenketten zu Kristalliten anlagern können. Je nach Packungsart wird die zugrunde liegende Kristallstruktur als "end-on" oder "interdigitating"-Kristallstruktur bezeichnet, wie in Figur 1 schematisch dargestellt.

Im Falle der dichter gepackten "interdigitating"-Kristallstruktur ragen die Seitenketten in die Zwischenräume der Seitenketten der Nachbarmoleküle und liegen somit parallel zu den benachbarten Seitenketten vor. Im Gegensatz dazu stehen die Seitenketten in der "end-on"-Kristallstruktur frontal zu den Seitenketten der Nachbarmoleküle orientiert. Die Kristallitdicke (d) ist im Falle der "interdigitating"-Kristallstruktur somit wesentlich kleiner als im Falle der "end-on"-Kristallstruktur. Im ideal gepackten Fall entspricht die Kristallitdicke für die "interdigitating"-Kristallstruktur (dᵢ) der Länge der Seitenkette. Die Kristallitdicke in der ideal gepackten "end-on"-Kristallstruktur (dₑ) entspricht der doppelten Länge der Seitenkette zuzüglich eines C-C Abstands zwischen den beiden Enden der frontal gepackten Seitenketten.

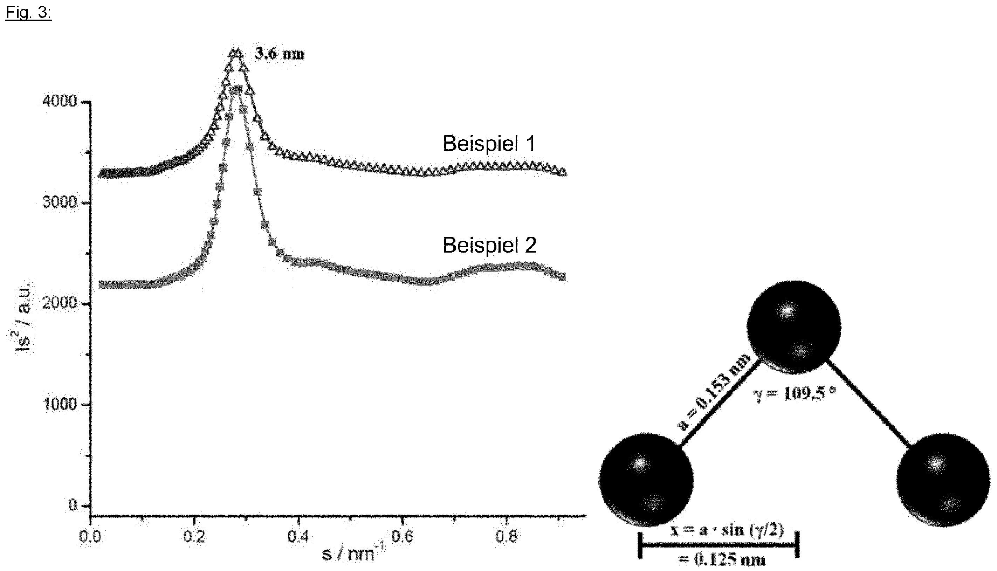

Die Untersuchung des zugrundeliegenden Kristallisationsmechanismus erfolgte mittels SAXS Messungen anhand der Beispiele 1 und 2. Figur 3 zeigt hierzu die Ergebnisse der durchgeführten SAXS Experimente.

Die ermittelten Abstände aus dem SAXS Diffraktogramm betragen im Fall der Beispiele 1 und 2 3,6 nm. Dies spricht gegen das Vorliegen einer "interdigitating"-Kristallstruktur und ist ein starkes Indiz dafür, dass die untersuchten Materialien bevorzugt im "end-on"-Mechanismus kristallisieren. Im Falle einer Bindungslänge von 0,153 nm und einem Tetraederwinkel von 109,5° beträgt der Abstand zweier C Atome entlang der Alkylseitenkette 0,125 nm. Tabelle 3 vergleicht die daraus berechneten idealen Kristallitdicken dᵢ und dₑ mit den gemessenen Gitterkonstanten aus dem Beugungsexperiment.

Es ist deutlich zu erkennen, dass die gemessenen Abstände (d_{gemessen}) zu groß für das Vorliegen einer "interdigitating"-Kristallstruktur sind, während die Werte sehr nahe an den theoretisch berechneten Werten für eine ideale "end-on"-Kristallisation liegen. Eine mögliche Erklärung hierfür ist, dass aufgrund der hohen Veresterungsgrade der untersuchten Proben nur wenig Platz zwischen den Seitenketten vorhanden ist. Somit können sich die Seitenketten benachbarter Moleküle nicht in die Zwischenräume einlagern, sondern orientieren sich frontal gegenüber den Fettsäureresten der benachbarten Polymerketten.

**Tabelle 3:**

| **Beispiel** | **dᵢ, ideal (nm)** | **dₑ, ideal (nm)** | **d_{gemessen} (nm)** |
|---|---|---|---|
| Beispiel 1 | 1,875 | 3,875 | 3,900 |
| Beispiel 2 | 1,875 | 3,875 | 3,900 |

### Rückstand der Haftmittel-Zusammensetzung nach Entfernen der Wundauflagen

Neben dem Aufschmelzen des statistischen Copolymers durch Körperwärme (ca. 37°C) wird mithilfe eines handelsüblichen Kühlakkus oder Eisbeutels (ca. 0°C) eine Kristallisation der Alkylester-Seitenketten des statistischen Copolymers eingeleitet. Dadurch steigt die Kohäsion der Haftmittel-Zusammensetzung stark an und diese löst sich weitgehend rückstandsfrei von der Haut des Patienten ab.

Dazu wurden Probenkörper aus einer handelsüblichen Zemuko^{®}-Wundkompresse ausgeschnitten (Abmessungen: 6,0 x 3,0 cm) und eine definierte Klebefläche (4,0 x 1,5 cm) gleichmäßig mit aufgeschmolzenem statistischen Copolymer (jeweils 300 mg) bestrichen. Die Proben wurden je 5 min bei 37°C unter Beschwerung mit einer Messingplatte (m = 2800 g) getempert und anschließend auf 0°C gekühlt, um anschließend Abzugstests durchführen zu können. Tabelle 4 zeigt hierzu die Ergebnisse der Abzugstests auf Aluminium. Der Haftmittelrückstand in Massen-% wurde durch das Gewicht der Proben vor und nach dem Abziehen ermittelt.

**Tabelle 4:**

| **Beispiel** | **Kühldauer (s)** | **Haftmittelrückstand auf Aluminium (Massen-%)** |
|---|---|---|
| Beispiel 1 | 120 | 0,00 ± 0,10 |
| Beispiel 2 | 120 | 0,00 ± 0,10 |

In diesem Zusammenhang wurde anhand von entsprechenden Abzugstests festgestellt, dass die Haftmittel-Zusammensetzung selbst einen starken Einfluss auf die Menge der Haftmittelrückstände bei den Abzugstests auf Aluminium haben. Je höher der Anteil an langkettigen Alkylester-Seitenketten, desto geringer ist der Haftmittelrückstand auf den untersuchten Oberflächen. Als Vergleichsbeispiel beträgt der Haftmittelrückstand von einem Leukoplast^{®}-Tape bei 37°C auf Aluminium 0,40 ± 0,13 Massen-%.

Die Erklärung für diesen Trend liegt in der Seitenketten-Kristallisation. Je höher der Anteil an Alkylester in der Haftmittel-Zusammensetzung ist, desto besser kann das Material kristallisieren. Die Kristallite wirken als physikalische Vernetzungspunkte und erhöhen dadurch die Kohäsion des Materials, was den Haftmittelrückstand reduziert. Auf eine Verlängerung der Kühldauer über 120 s wurde unter Berücksichtigung des Patientenwohls bei der späteren Anwendung verzichtet.

### Schälfestigkeit

Um die Klebrigkeit der hergestellten Beispiele 1 und 2 quantifizieren zu können, wurden in einem Abzugswinkel von 180° Abzugstests nach "ASTM D3330 Test Method A 180 Degree Peel Test" durchgeführt. Hierbei wird mit Hilfe einer Zug/Dehnungs-Maschine die Abzugskraft während des Abziehens gemessen. Nach einer kurzen Anlaufphase geht diese in ein Plateau über und wird als Abzugskraft in N/10 mm angegeben.

Um die Ergebnisse besser einordnen zu können, wurde handelsübliches Leukoplast^{®}-Tape als Referenz vermessen. Die Ergebnisse im vorigen Abschnitt zeigten, dass bei einer Kühldauer von 120 s der Haftmittelrückstand auf Aluminium nach dem Entfernen der Wundauflagen lediglich sehr gering vorliegt. Daher erfolgten alle 180° Abzugstests durch Abziehen der Wundauflagen von Edelstahlplatten nach Tempern für 5 min bei 37°C sowie nach Kühlen für 120 s bei 0°C.

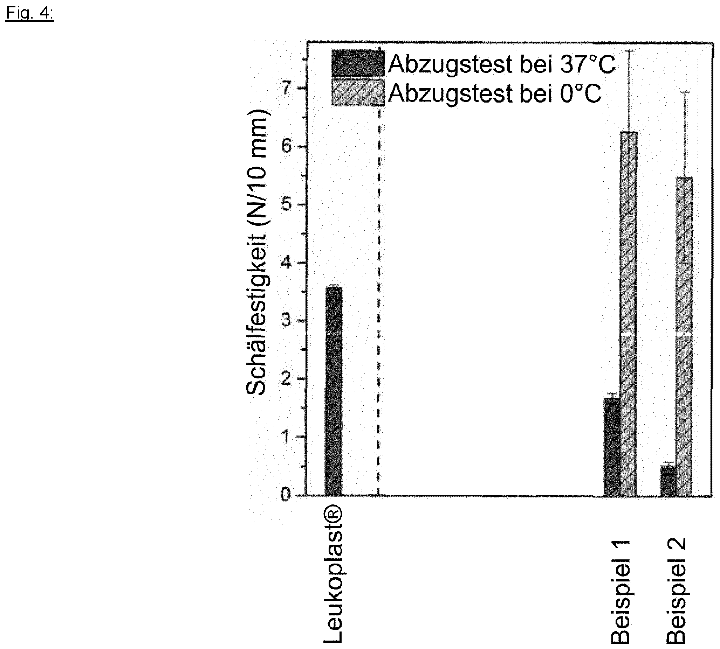

Die Abmessungen der Proben (handelsübliche Zemuko^{®}-Wundkompressen) betrugen dabei je 200 x 30 mm mit einer Klebefläche von 80 x 15 mm, welche mit 600 mg aufgeschmolzener Haftmittel-Zusammensetzung gleichmäßig bestrichen wurden. Während des Temperns bei 37°C wurden die Proben mit einem Gewicht von 2800 g beschwert, um das Anpressen der Kompressen auf die Wunde durch die Hand des Patienten zu simulieren. Die resultierenden thermoresponsiven Schälfestigkeiten der Beispiele 1 und 2 aus den Abzugstests bei 37°C und 0°C im Vergleich zu handelsüblichem Leukoplast^{®}-Tape bei 37°C sind in Figur 4 dargestellt.

Bei 37°C wird zum Ablösen der Wundauflagen von der Edelstahlplatte bei Beispielen 1 und 2 eine geringere Abzugskraft benötigt als bei Leukoplast^{®}. Im Speziellen liegt die Schälfestigkeit für die Beispiele 1 und 2 bei ca. 15 bis 47% im Vergleich zur Referenz Leukoplast^{®}. Dadurch eignen sich diese Haftmittel-Zusammensetzungen bei 37°C für die Anwendung als Haftmittel-Zusammensetzung für thermoresponsive Wundauflagen.

Neben der zuverlässigen Haftung bei Körpertemperatur ist das rückstandsfreie Ablösen bei möglichst geringen Abzugskräften jedoch ebenfalls von Bedeutung. Die Abzugstests bei 0°C zeigen dabei einen starken Einfluss der Haftmittel-Zusammensetzung auf die Schälfestigkeit. Je höher der Anteil an Vinylalkylester im statistischen Copolymer ist, desto geringer ist die benötigte Abzugskraft. Es zeigte sich, dass bei den Beispielen 1 und 2 ein schmerzfreies Ablösen bei 0°C realisierbar ist.

### Biokompatibilität

Da die hergestellten statistischen Vinylalkohol/Vinylalkylester-Copolymere als thermoresponsives Haftmittel auf der menschlichen Haut angewendet werden sollen, spielt die Biokompatibilität der verwendeten Materialien eine große Rolle. Um sicherzustellen, dass während der Anwendung keine toxischen Bestandteile aus dem Material heraus diffundieren, wurden deshalb Eluatversuche zur Evaluierung der Biokompatibilität nach ISO 10993-05 durchgeführt.

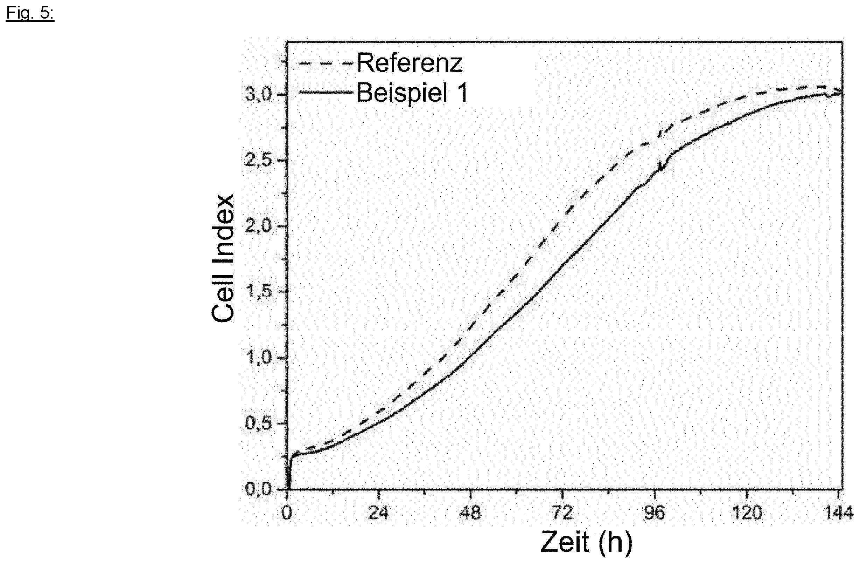

Dazu wurden die statistischen Vinylalkohol/Vinylalkylester-Copolymere für sieben Tage bei 37°C in Zellkulturmedium eingelegt und anschließend eine Zellkultivierung mit dem Medium, in welchem die Proben zuvor eingelegt worden waren, durchgeführt. Die Durchführung dieser Eluatversuche erfolgte anhand von HaCaT Zellen der Firma "CLS Cell Lines Service". Das Zellwachstum wurde dabei über mehrere Tage mittels Impedanzmessung verfolgt und in Form des "Cell Index" ausgewertet. Durch Vergleich mit unbehandeltem Zellkulturmedium als Referenz kann somit darauf geschlossen werden, ob aus dem Material zellwachstumshemmende Stoffe heraus migrieren. In Figur 5 ist der Cell Index gegen die Zeit für Beispiel 1 und die Referenz aufgetragen. Die nahezu identischen Kurvenverläufe der jeweiligen Probe mit der Referenz in Figur 5 zeigen, dass das Zellwachstum durch Kontakt mit Beispiel 1 kaum beeinflusst wurde. Es konnte somit sichergestellt werden, dass über einen Zeitraum von sieben Tagen keine zellwachstumshemmenden Substanzen aus den Materialien in das Medium diffundieren. Dieses Ergebnis steht für eine gute Biokompatibilität der Copolymere, was als Grundvoraussetzung für die Anwendung auf der menschlichen Haut gilt.

## Patentansprüche

1. Thermoresponsive Wundauflage mit thermisch schaltbarer Haftung/Enthaftung, umfassend:
ein Trägermaterial, und
eine Haftmittel-Zusammensetzung, aufgebracht auf einer Oberfläche des Trägermaterials, wobei die Haftmittel-Zusammensetzung umfasst:
mindestens ein Vinylalkohol/Copolymer mit der nachstehenden Formel (I): 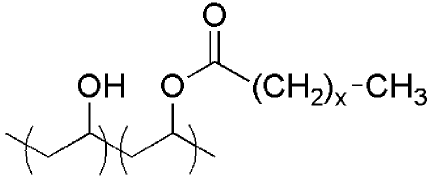
wobei x bezüglich jeder Fettsäureeinheit unabhängig voneinander eine ganze Zahl von 10 bis 18 beträgt,
wobei das Copolymer gemäß Formel (I) bei Körpertemperatur als Schmelze vorliegt, die auf Wunden und Haut haftet, und beim Abkühlen auf eine Temperatur oberhalb des Gefrierpunkts von Wasser die Kristallisation des Copolymers gemäß Formel (I) die Enthaftung der Wundauflage bewirkt.

2. Thermoresponsive Wundauflage nach Anspruch 1, wobei das mindestens eine Copolymer gemäß Formel (I) eine Schmelztemperatur (Tₘ) von 20 bis 35°C und eine Schmelzenthalpie (ΔHₘ) von 20 J/g oder mehr aufweist.

3. Thermoresponsive Wundauflage nach Anspruch 1 oder 2, wobei das mindestens eine Copolymer gemäß Formel (I) eine zahlengemittelte Molmasse (Mₙ) von 1000 bis 100000 g/mol aufweist, wobei Mₙ durch Gel-Permeations-Chromatographie (GPC) bestimmt wird.

4. Thermoresponsive Wundauflage nach einem der Ansprüche 1 bis 3, wobei das mindestens eine Copolymer gemäß Formel (I) einen Polydispersitätsindex (PDI) von 1,0 bis 3,0 aufweist, wobei der PDI durch Gel-Permeations-Chromatographie (GPC) bestimmt wird.

5. Thermoresponsive Wundauflage nach einem der Ansprüche 1 bis 4, wobei die Haftmittel-Zusammensetzung das mindestens eine Copolymer gemäß Formel (I) in einer Menge im Bereich von 40 bis 100 Gew.-% umfasst.

6. Thermoresponsive Wundauflage nach einem der Ansprüche 1 bis 5, wobei die Haftmittel-Zusammensetzung weiter mindestens eine antimikrobielle Substanz umfasst.

7. Thermoresponsive Wundauflage nach einem der Ansprüche 1 bis 6, wobei x 12 beträgt und ein molares Verhältnis von Vinylalkohol-Einheiten zu Vinylfettsäureester-Einheiten des mindestens einen Copolymers gemäß Formel (I) 0,05 bis 0,50, vorzugsweise 0,06 bis 0,40, bestimmt mittels ¹H-NMR-Spektroskopie, beträgt.

8. Thermoresponsive Wundauflage nach einem der Ansprüche 1 bis 7, wobei das mindestens eine Copolymer gemäß Formel (I) ein statistisches Copolymer ist.

9. Thermoresponsive Wundauflage nach einem der Ansprüche 1 bis 8, wobei das mindestens eine Copolymer gemäß Formel (I) weiter ein oder mehrere Einheiten, abgeleitet von Monomeren, ausgewählt aus Ethylen und Vinylacetat, umfasst.

10. Thermoresponsive Wundauflage nach einem der Ansprüche 1 bis 9, wobei das Trägermaterial aus mindestens einem Material, ausgewählt aus der Gruppe, bestehend aus Zellulose, Polyvinylchlorid, Polyethylen, Polyethylenterephthalat, Polyurethan und Polyetherester, besteht.

11. Verfahren zur Herstellung einer thermoresponsiven Wundauflage nach einem der Ansprüche 1 bis 10, wobei das Trägermaterial mit einer Schmelze, Lösung oder Dispersion von mindestens einem Copolymer gemäß Formel (I), wie vorstehend definiert, beschichtet wird.

## Claims

1. A thermoresponsive wound dressing with thermally switchable adhesion/detachment, comprising:
a carrier material, and
an adhesive composition applied to a surface of the carrier material, the adhesive composition comprising:
at least one vinyl alcohol/copolymer with the following formula (I): 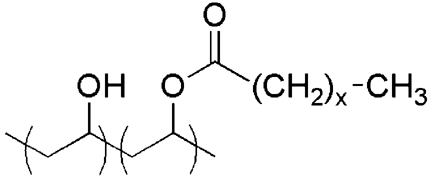
where x is an integer from 10 to 18 independently of one another with respect to each fatty acid unit,
where the copolymer according to formula (I) is present at body temperature as a melt that adheres to wounds and skin, and upon cooling to a temperature above the freezing point of water, the crystallization of the copolymer according to formula (I) causes the wound dressing to detach.

2. The thermoresponsive wound dressing according to claim 1, wherein the at least one copolymer according to formula (I) has a melting temperature (Tₘ) of 20 to 35°C and a melting enthalpy (ΔHₘ) of 20 J/g or more.

3. The thermoresponsive wound dressing according to claim 1 or 2, wherein the at least one copolymer according to formula (I) has a number-average molar mass (Mₙ) of 1000 to 100,000 g/mol, wherein Mₙ is determined by gel permeation chromatography (GPC).

4. The thermoresponsive wound dressing according to one of claims 1 to 3, wherein the at least one copolymer according to formula (I) has a polydispersity index (PDI) of 1.0 to 3.0, wherein the PDI is determined by gel permeation chromatography (GPC).

5. The thermoresponsive wound dressing according to one of claims 1 to 4, wherein the adhesive composition comprises the at least one copolymer according to formula (I) in an amount in the range from 40 to 100 wt.%.

6. The thermoresponsive wound dressing according to one of claims 1 to 5, wherein the adhesive composition further comprises at least one antimicrobial substance.

7. The thermoresponsive wound dressing according to one of claims 1 to 6, wherein x is 12 and a molar ratio of vinyl alcohol units to vinyl fatty acid ester units of the at least one copolymer according to formula (I) is 0.05 to 0.50, preferably 0.06 to 0.40, determined by means of ¹H-NMR spectroscopy.

8. The thermoresponsive wound dressing according to one of claims 1 to 7, wherein the at least one copolymer according to formula (I) is a statistical copolymer.

9. The thermoresponsive wound dressing according to one of claims 1 to 8, wherein the at least one copolymer according to formula (I) further comprises one or more units derived from monomers selected from ethylene and vinyl acetate.

10. The thermoresponsive wound dressing according to one of claims 1 to 9, wherein the carrier material consists of at least one material selected from the group consisting of cellulose, polyvinyl chloride, polyethylene, polyethylene terephthalate, polyurethane and polyether ester.

11. A method for producing a thermoresponsive wound dressing according to one of claims 1 to 10, wherein the carrier material is coated with a melt, solution or dispersion of at least one copolymer according to formula (I), as defined above.

## Revendications

1. Pansement thermoréactif avec adhésion/décollage pouvant être commuté(e) thermiquement, comprenant :
un matériau support, et
une composition adhésive appliquée à une surface du matériau support, la composition adhésive comprenant :
au moins un copolymère d'alcool vinylique avec la formule suivante (I) : 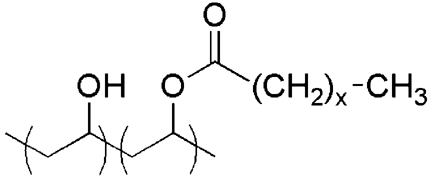
où x est un entier allant de 10 à 18 indépendamment l'un de l'autre par rapport à chaque unité d'acide gras,
où le copolymère selon la formule (I) est présent à la température corporelle en tant que matière fondue qui adhère à des plaies et la peau, et dès le refroidissement à une température au-dessus du point de congélation de l'eau, la cristallisation du copolymère selon la formule (I) provoque le décollage du pansement.

2. Pansement thermoréactif selon la revendication 1, dans lequel l'au moins un copolymère selon la formule (I) a une température de fusion (Tₘ) de 20 à 35 °C et une enthalpie de fusion (ΔHₘ) de 20 J/g ou plus.

3. Pansement thermoréactif selon la revendication 1 ou 2, dans lequel l'au moins un copolymère selon la formule (I) a une masse molaire moyenne en nombre (Mₙ) de 1000 à 100 000 g/mol, dans lequel Mₙ est déterminée par chromatographie d'exclusion diffusion (GPC).

4. Pansement thermoréactif selon une des revendications 1 à 3, dans lequel l'au moins un copolymère selon la formule (I) a un indice de polydispersité (PDI) de 1,0 à 3,0, dans lequel le PDI est déterminé par chromatographie d'exclusion diffusion (GPC).

5. Pansement thermoréactif selon une des revendications 1 à 4, dans lequel la composition adhésive comprend l'au moins un copolymère selon la formule (I) en une quantité dans la plage allant de 40 à 100 % en poids.

6. Pansement thermoréactif selon une des revendications 1 à 5, dans lequel la composition adhésive comprend en outre au moins une substance antimicrobienne.

7. Pansement thermoréactif selon une des revendications 1 à 6, dans lequel x est 12 et un rapport molaire d'unités d'alcool vinylique sur des unités d'ester d'acide gras vinylique de l'au moins un copolymère selon la formule (I) est de 0,05 à 0,50, de préférence 0,06 à 0,40, déterminé au moyen d'une spectroscopie RMN ¹H.

8. Pansement thermoréactif selon une des revendications 1 à 7, dans lequel l'au moins un copolymère selon la formule (I) est un copolymère statistique.

9. Pansement thermoréactif selon une des revendications 1 à 8, dans lequel l'au moins un copolymère selon la formule (I) comprend en outre une ou plusieurs unités dérivées de monomères sélectionnés parmi l'éthylène et l'acétate de vinyle.

10. Pansement thermoréactif selon une des revendications 1 à 9, dans lequel le matériau support est constitué d'au moins un matériau sélectionné parmi le groupe constitué de la cellulose, du chlorure de polyvinyle, du polyéthylène, du polyéthylène téréphtalate, du polyuréthane et du polyéther ester.

11. Procédé de production d'un pansement thermoréactif selon une des revendications 1 à 10, dans lequel le matériau support est enduit d'une matière fondue, solution ou dispersion d'au moins un copolymère selon la formule (I), telle que définie ci-dessus.
